# EUROPEAN PATENT SPECIFICATION

(11) **EP 0 355 793 B1**
(45) Date of publication and mention of the grant of the patent: **10.07.1996**
(21) Application number: 89115469.2
(22) Date of filing: 22.08.1989
(51) Int. Cl.: C07K 7/00, C07K 7/08, A61K 38/00

(54) **Neuropeptide Y agonists and partial agonists**
Neuropeptid Y-Agonisten und partielle Agonisten
Agonistes de neuropeptide y et agonistes partiels

(30) Priority: 26.08.1988 US 237591
(43) Date of publication of application: 28.02.1990
(73) Proprietor: MERRELL PHARMACEUTICALS INC., Cincinnati,Ohio 45215-6300 (US)
(72) Inventor: Krstenansky, John L., CincinnatiOhio 45208 (US)
(74) Representative: VOSSIUS & PARTNER

(56) References cited:
- DE-A- 3 811 193
- PROC. NATL. ACAD. SCI. USA, vol. 79, September 1982, pages 5485-5489, Washington, DC, US; K. TATEMOTO: "Neuropeptide Y: Complete amino acid sequence of the brain peptide
- FEBS LETTERS, vol. 244, no. 1, 13th February 1989, pages 119-122, Amsterdam, NL; A. BECK et al.: "Highly potent and small neuropeptide Y agonist obtained by linking NPY 1-4 via spacer to alpha-helical NPY 25-36"
- PROC. NATL. ACAD. SCIENCE, USA, vol. 86, June 1989, pages 4377-4381, Washington, DC, US; J.L. KRSTENANSKY et al.: "Centrally truncated and stabilized porcine neuropeptide Y analogs: Design, synthesis, and mouse brain receptor binding"

## Description

This invention relates to novel peptide derivatives which are agonists of neuropeptide Y

Porcine neuropeptide Y (pNPY) is a 36 amino acid residue peptide that belongs to a unique family of peptides having a wide distribution throughout the central and peripheral nervous systems. Receptors for NPY are found in the central nervous system and in the periphery. In the brain, NPY is a potent stimulator of food intake, stimulates leutinizing hormone, growth hormone and prolactin, and produces cardiovascular depression. NPY is also a potent peripheral vasoconstrictor and has been reported tc cause transient myocardial ischaemia in patients with angina pectoris. Agents which are agonists of these receptors are expected to increase appetite, decrease sexual behavior, decrease thyroid stimulating hormone, prolactin, leutenizing hormone and therefore would be useful as contraceptive agents, to diminish sex drive in sex offenders, and in the treatment of reproductive-system related disorders, such as precocious puberty, endometriosis, breast tumors, prostate tumors, and decrease growth hormone levels by stimulating release and to act as peripheral vasodilators and therefore act as hypotensive agents. The compounds of this invention could be used in the treatment of eating disorders such as anorexia nervosa.

Novel peptide derivatives of formulae 1 - 3 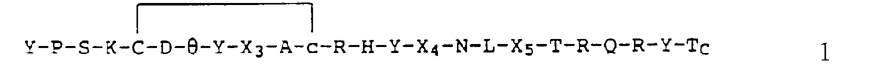 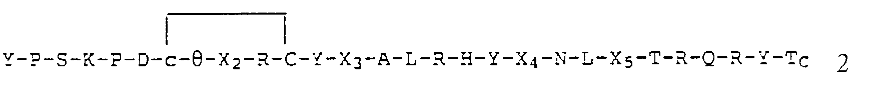 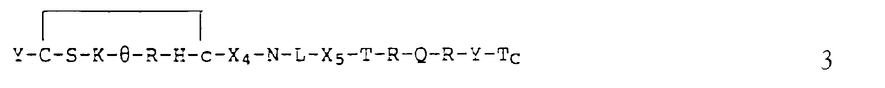 wherein
X₂ is S or A;
X₃ is S or A;
X₄ is L, I, M, Nle, or V;
X₅ is L, I, M, Nle, or V;
Tc is OR or NHR';
wherein R' is a hydrogen or a (C₁-C₄)alkyl group
6 is a group of the structural formula

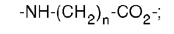

wherein n is an integer of from 1-11, and the pharmaceutically acceptable salts thereof are agonists of neuropeptide Y These peptide derivatives increase blood pressure in warm blooded animals and are also useful in the treatment of eating disorders such as anorexia nervosa.

The following common abbreviations of the amino acids and amino and carboxy terminal groups are used throughout this specification:

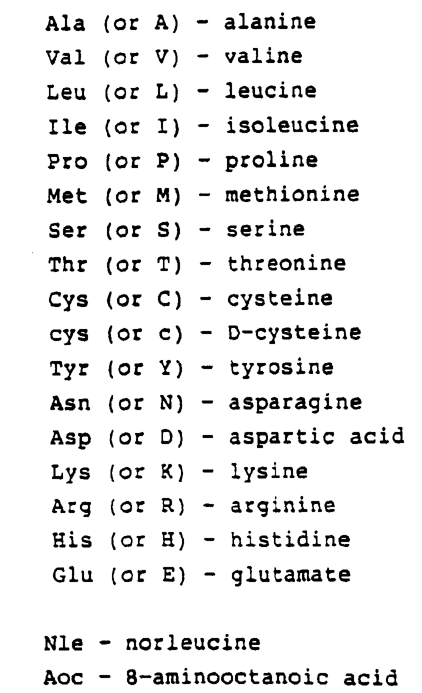

An alkyl group is taken to include straight, branched, or cyclic alkyl groups, for example, methyl, ethyl, propyl, isopropyl, butyl, isobutyl, tert-butyl, pentyl, isopentyl, sec pentyl, cyclopentyl, hexyl, isohexyl, cyclohexyl and cyclopentylmethyl. An acyl group of from 2 to 10 carbon atoms is taken to include straight, branched, cyclic, saturated and unsaturated acyl groups having 1 or 2 carbonyl moieties per group, for example, acetyl, benzoyl succinyl, maleyl, and glutaryl. Those peptides wherein the amino group of the aminc terminal amino acid is substituted with two alkyl or acyl groups are also considered to be within the scope of the peptides of this invention.

The natural amino acids, with the exception of glycine, contain a chiral carbon atom. Unless otherwise specifically indicated, the optically active amino acids, referred to herein, are of the L-configuration.

The polypeptides of formula 1 can form pharmaceutically acceptable salts with any non-toxic, organic or inorganic acid Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-ethenamine, N,N'-dibenzylethylenediamine, dihy- droabietylamine, N-(lower)alkylpiperidine, and any other suitable amine.

As with any generic group of chemical compounds, certain groups are preferred Applicants prefer those peptide derivatives of formula 1 wherein X₂ and X₃ are independently serine (S) or alanine (A), as well as those peptide derivatives of formula 1 wherein X₄ or X5 are independently leucine (L) or isoleucine (I) wherein Tc is NH₂ and wherein 8 is Aoc. The most preferred peptide derivatives of formula 1 - 3 are the peptide derivatives of formula 5 - 7 respectively

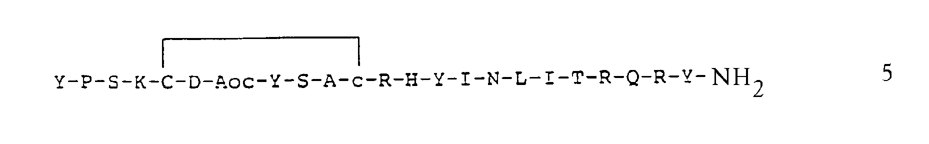

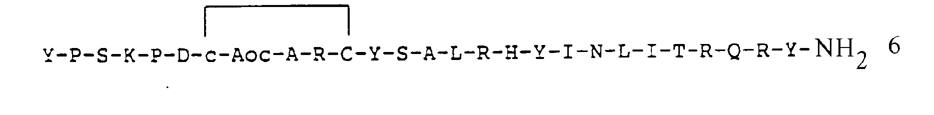

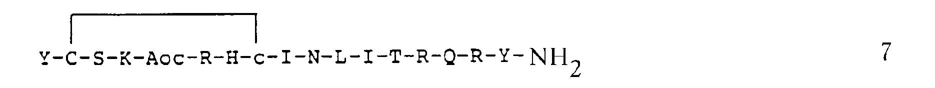

The proteins of this invention can be prepared by a variety of procedures readily known to those skilled in the art. Such procedures include the solid phase sequential procedure which can be performed using established automated methods such as by use of an automated peptide synthetizer.

The resin support employed can be any suitable resin conventionally employed in the art for the solid phase preparation of polypeptides, preferably polystyrene which has been cross-linked with from 0.5 to about 3 percent divinyl benzene, which has been either converted to the p-mechylbenzhydrylamine or benzhydrylamine derivative (for C-terminal amides) or chloromethylated or hydroxymethylated to provide sites for ester formation with the initially introduced a-amino protected amino acid (for producing C-terminal alkylamides and esters).

An example of a hydroxymethyl resin is described by Bodanszky, et aI., Chem. Ind. (London) 38, 1597-98 (1966). A chloromethylated resin is commercially available from Bio Rad Laboratories, Richmond, California, and the preparation of such a resin is described by Stewart et al., "Solid Phase Peptide Synthesis" (Freeman & Co., San Francisco 1969), Chapter 1, pp. 1-6. The protected amino acid can be bound to the resin by the procedure of Gisin, Helv. Chem Acta, 56, 1476 (1973). Many resin bound, protected amino acids are commercially available. As an example, to prepare a polypeptide of this invention wherein the carboxy terminal end is a Th r residue, a tert-butyloxycarbonyl (Boc) protected Thr bound to a benzylated, hydroxymethylated phenylacetamidomethyl (PAM) resin can be used and is commercially available.

Following the coupling of the a-amino protected amino acid to the resin support, the protecting group is removed using any suitable procedure such as by using trifluoroacetic acid in methylene chloride, trifluoroacetic acid alone, or HCI in dioxane. The deprotection is carried out at a temperature of between 0°C and room temperature. Other standard cleaving reagents and conditions for removal of specific a-amino protecting groups may be used. After removal of the a-amino protecting group the other amino protected amino acids are coupled step-wise in the desired order. Alternatively, multiple amino acid groups may be coupled by the solution method prior to coupling with the resin supported amino acid sequence.

The a-amino protecting group employed with each amino acid introduced into the polypeptide sequence may be any such protecting group known to the art. Among the classes of a-amino protecting groups contemplated are (1) acyl type protecting groups such as: formyl, trifluoroacetyl, phthalyl, toluenesulfonyl (tosyl), benzenesulfonyl, nitro- phenylsulfolnyl, tritylsulfonyl, o-nitrophenoxyacetyl and a-chlorobutyryl; (2) aromatic urethan type protecting groups such as benzyloxycarbonyl and substituted benzyloxycarbonyl, such as p-chlorobenzyloxycarbonyl, p-nitrobenzyloxy-carbonyl, p-bromobenzyl-oxycarbonyl, p-methoxybenzyloxycarbonyl, 1-(p-biphenylyl)-1-methylethoxycarbonyl, a, a-dimethyl-3,5-dimethoxybenzyloxycarbonyl, benzhydryloxycarbonyl and 9-fluorenylmethoxycarbonyl (Fmoc); (3) aliphatic urethan protecting groups such as tert-butyloxycarbonyl (Boc), diisopropylmethoxycarbonyl, isopropyloxycarbonyl, ethoxycarbonyl and allyloxycarbonyl; (4) cycloalkyl urethan type protecting groups such as cyclopentyloxycarbonyl, adamantyloxycarbonyl and cyclohexyloxycarbonyl; (5) thio urethan type protecting groups such as phenylthio- carbonyl; (6) alkyl type protecting groups such as triphenylmethyl (trityl) and benzyl, and (7) trialkylsilane groups such as trimethylsilane. The preferred a-amino protecting group is tert-butyloxycarbonyl or Fmoc.

The selection of an appropriate coupling reagent is within the skill of the art. A particularly suitable coupling reagent where the amino acid to be added is Gln, Asn or Arg is N,N'-diisopropylcarbodiimide and I-hydroxy-benzotriazole. The use of these reagents prevents nitrile and lactam formation. Other coupling agents are (1) carbodiimides (e.g., N,N'- dicyclohexylcarbodiimide and N-ethyl-N'-(y-dimethylaminopropylcarbodiimide); (2) cyanamides (e.g., N,N-dibenzylcy- anamide); (3) ketenimines; (4) isoxazolium salts (e.g., N-ethyl-5-phenyl-isoxazolium-3'-suifonate; (5) monocyclic nitrogen containing heterocyclic amides of aromatic character containing one through four nitrogens in the ring such as imidazolides, pyrazolides, and 1,2,4-triazoiides. Specific heterocyclic amides that are useful include N,N'-carbonyldiimidazole and N,N-carbonyl-di-1,2_{;}4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., (Boc-Ala)2-O) and (8) nitrogen containing heterocyclic compounds having a hydroxy group on one ring nitrogen (e.g., N-hydroxyphthalimide, N-hydroxysuccinimide and 1-hydroxybenzotriazole). Other activating reagents and their use in peptide coupling are described by Kapoor, J. Pharm. Sci., 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the a-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

After the desired amino acid sequence has been obtained, the peptide is removed from the resin. This can be done by hydrolysis such as by treatment of the resin bound polypeptide with a solution of dimethyl sulfide, p-cresol and thiocresol in liquid hydrofluoric acid.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed during cleavage of the protecting group of the a-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxy-carbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The carboxylic group of Aspartic acid and Glutamic acid can be protected with a benzyl or cyclohexyl group. The preferred protecting group is benzyl.

These groups can be removed by procedures well known in the art. Typically protecting group removal is done after the peptide chain synthesis is complete but the protecting groups can be removed at any other appropriate time.

The ability of the peptide derivatives of formula 1 to act as agonists of neuropeptide Y can be demonstrated by the ability of such peptides to compete with iodinated neuropeptide Y for receptors using the method of Lundberg et al. Eur. J. Pcol. 145:21-9 (1988). ¹²⁵l-Bolton-Hunter-neuropeptide Y (BHNPY; Amersham) binding was carried out in porcine spleen crude membranes. Membranes from frozen spleen were prepared as described previously fortachykinin peptide binding studies (Buck et al., 1984). An aliquot of membrane preparation (approximately 15 mg tissue) was incubated at room temperature for 2 hr in buffer (pH 7.4) containing the peptide analog, 130 mM NaCI, 2.7 mM KCI, 2 mM MgCl₂, 1.8 mM CaCl₂, 20 mM HEPES, 4 mg/ml BSA, 40 µg/ml bacitracin, 4 µg/ml leupeptin and 4 µg/ml chymosta- tin. BHNPY was included in a concentration of 0.1 nM and non-specific binding was determined by the inclusion of 1 µm pNPY Samples were rapidly filtered over Whatman GF/C filters presoaked overnight in 0.5% histone (type II-AS; Sigma) and washed two times with ice-cold, plain HEPES-salt buffer (pH 7.4). lC₅₀ values for test peptides were calculated from 6 to 10 point competition curves. Utilizing this procedure the peptide derivatives of Examples 1 and 2 were found to have an IC₅₀ of < 50nM.

By virtue of the ability of the peptide derivatives of this invention to act as agonists of neuropeptide Y the compounds possess valuable pharmacologic properties such as hypertensive activity as well as vasoconstricting activity and constricting of the coronary artery, colon relaxing activity, and gastric emptying diminution. Significant medical uses of the NPY agonists of this invention are in the treatment of eating disorder such as anorexia nervosa.

The dose of a peptide derivative of this invention required to agonize neuropeptide Y and therefore produce a hypertensive or vasoconstricting and other effects is from 0.2 mg/kg to 250 mg/kg of patient body weight per day depending on the patient, the severity of the condition to be treated and the peptide derivative selected. The suitable dose for a particular patient can be readily determined. Preferably from 1 to 4 daily doses would be administered typically with from 5 mg to 100 mg of active compound per dose.

The term "patient" used herein is taken to mean mammals such as primates, including humans, sheep, horses, cattle, pigs, dogs, cats, rats and mice.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol can containing a peptide derivative of this invention in a spray or dry powder form.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

### EXAMPLES

This invention is illustrated by the following, nonlimiting examples.

### EXAMPLE 1

Preparation of

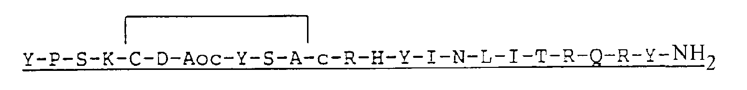

The title peptide derivative was synthesized on a 0.5 mmol scale by solid-phase methods on p-methylbenzhydr- ylamine resin (0.40 mmol/g; Peptides Intl.) using an Applied Biosystems Model 430-A Peptide Synthesizer. All residues were double coupled as the symmetrical anhydrides of the N^{α}-t-Boc-protected amino acids with the exception of Arg, Asn and Gln which were double coupled by the DCC/HOBT methodology The side chain protection was as follows: Arg(Tos), Asp(Chx), Cys(pMeBzl), Glu(Bzl), His(Tos), Ser(Bzl), Tyr(2-BrZ), Thr(Bzl), Lys(2-CIZ). The peptides (0.25 mmol theory) were cleaved from the resin support and deprotected in liquid HF containing 5% anisole at -5°C for 40 min. After removal of the HF in vacuo the peptide was extracted from the resin with 30% acetic acid and water. The extract was diluted to 1 liter, the pH adjusted to between 8 and 9 with ammonium hydroxide and 0.01 N potassium ferricyanide was added until a yellow color persisted (approx. 25 ml). After stirring for 30 min, the pH was lowered to <5 with glacial acetic acid and the solution was stirred with 25 ml of settled AC 3 X4A resin (Bio Rad) for 2 hours The solution was filtered from the resin and lyophilized. The peptidic material that remained was purified by preparative HPLC on a Dynamax C-18 column (41.4 x 250 mm; Rainin) using acetonitrile in 0.1 % trifluoroacetic acid as an eluant. The purity and identity of the peptide were assessed by analytical HPLC (Vydac 218TP54 column, 4.6 x 250 mm, 2.0 ml/min, t_{c}- 1.9 min, linear gradient of 15-40% acetonitrile in 0.1% TFA over 25 min), amino acid analysis (AAA)(6 N HCI containing 8% phenol; 106°C; 20 and 40 hr), and fast atom bombardment-mass spectrometry (FAB-MS)(M-Scan Ltd.).

AAA^{a}: B-1.89; T-0.99; S-1.68; Z-1.13; P-0.93; A-1.03; L-1.09; 1-2.07; Y-3.88; H-0.94; R-3.06.
^{a}6N HCI, 24 Hr, 106°C.
FAB-MS 2888.0 ± 1 mu.

### EXAMPLE 2

Preparation of

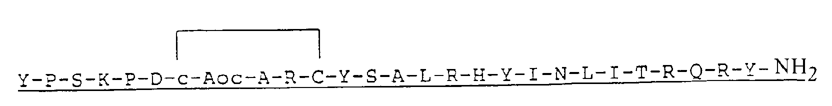

Using substantially the procedure of Example 1, the title compound was prepared.

AAA^{a}: B-2.03; T-1.05; S-1.83; Z-1.10; P-1.98; A-2.04; L-2.07; I-1.93;Y-3.91; K-1.02; H-0.98; R-3.87.
^{a}6N HCI, 24 Hr, 106°C.
FAB-MS 3327 ± 1 mu.

### EXAMPLE 3

Preparation of

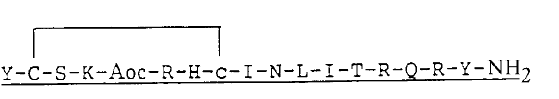

Using substantially the procedure of Example 1, the title compound was prepared.

AAA^{a}: B-1.00; S-1.00; T-1.01; Z-1.03; L-0.99; l-1.86; Y-2.06; H-0.97; R-2.97; K-1.10.
^{a}6N HCI, 24 Hr, 106°C.
FAB-MS 2193 ± mu.

In a like manner compounds of Examples 4 - 6 are prepared

Ex.

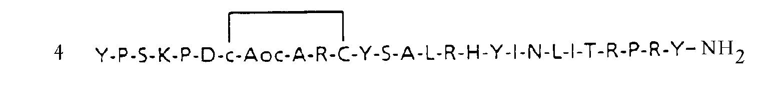

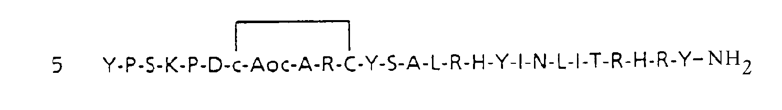

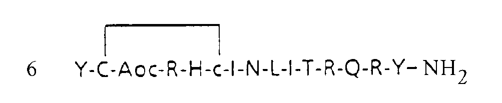

Compounds of examples 4 - 6 have the following characteristics :

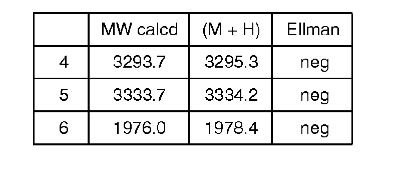

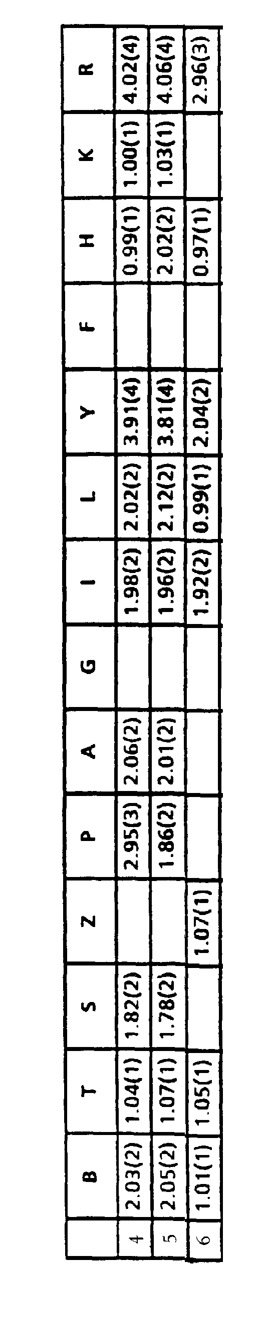

## Claims (Claims for the following Contracting State(s) : AT, BE, CH, DE, FR, GB, IT, LI, LU, NL, SE)

1. A peptide derivative of the formulae 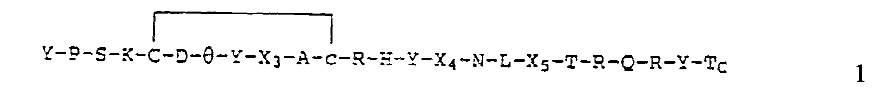 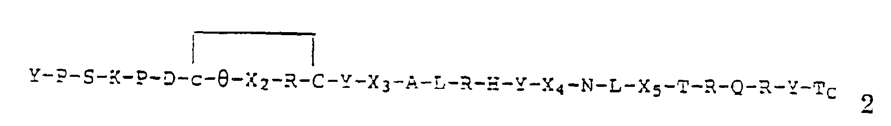 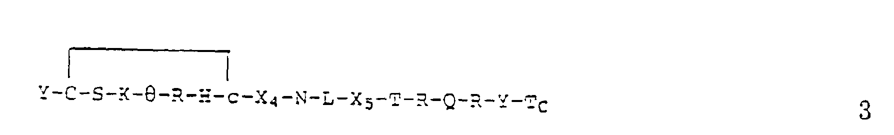 wherein
X₂ is S or A;
X₃ is S or A;
X₄ is L, l; M, Nle, or V;
X_{S} is L, l; M, Nle, or V;
Tc is OR' or NHR';
wherein R' is a hydrogen or a (C₁-C₄) alkyl group;
θ is a group of the structural formula 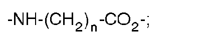
wherein n is an integer of from 1-11
or a pharmaceutically acceptable salt thereof

2. The peptide derivative of claim 1 wherein X₂ is A.

3. The peptide derivative of claim 1 or 2 wherein X₃ is S.

4. The peptide derivative of any one of claims 1 to 3 wherein X₄ is l.

5. The peptide derivative of any one of claims 1 to 4 wherein X₅ is l.

6. The peptide derivative of any one of claims 1 to 5 wherein θ is Aoc.

7. The peptide derivative of any one of claims 1 to 6 which is 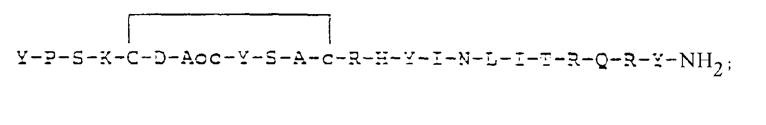 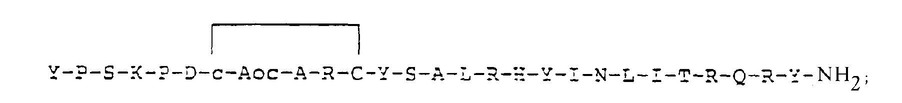 or

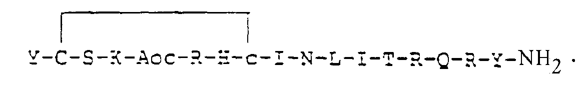

8. A process for preparing a peptide derivative according to any one of claims 1 to 7 which comprises binding a suitably protected tyrosine to an activated resin support, subsequently binding the other alpha amino protected amino acids from proline or cysteine to the carboxy terminal tyrosine to the terminal amino group of the growing peptidic chain which has meanwhile been exposed by removing its amino protecting groups, where an internally cyclized peptide derivative is desired subjecting the linear peptide to an oxidative coupling, and finally isolating the desired peptide or a pharmaceutically acceptable salt thereof.

9. A peptide derivative or a pharmaceutically acceptable salt thereof according to any one of claims 1 to 7 or a mixture thereof for use as a pharmaceutically active substance.

10. A peptide derivative of formula 3 or pharmaceutically acceptable salt thereof according to any one of claims 1 to 7 or a mixture thereof for the treatment of hypotension.

11. A peptide derivative or a pharmaceutically acceptable salt thereof according to any one of claims 1 to 7 or a mixture thereof for the treatment of eating aversion disorders.

12. A peptide derivative or a pharmaceutically acceptable salt thereof according to any one of claims 1 to 7 or a mixture thereof for use as an activator of a neuropeptide Y receptor.

## Claims (Claims for the following Contracting State(s) : ES, GR)

1. A peptide derivative of the formulae 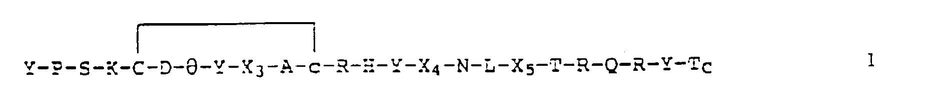 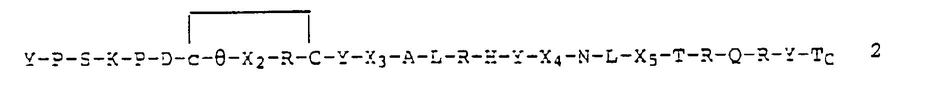 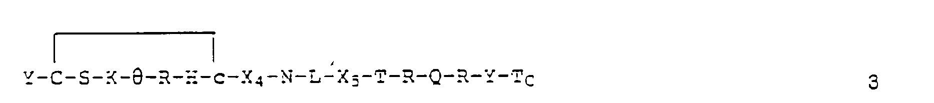 wherein
X₂ is S or A;
X₃ is S or A;
X₄ iS L, I, M, Nle, or V;
X₅ is L, l; M, Nle, or V;
Tc is OR' or NHR';
wherein R' is a hydrogen cr a (C₁-C₄) alkyl group;
8 is a group of the structural formula 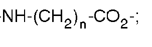
wherein n is an integer of from 1-11
or a pharmaceutically acceptable salt thereof.

2. The peptide derivative of claim 1 wherein X₂ is A.

3. The peptide derivative of claim 1 or 2 wherein X₃ is S.

4. The peptide derivative of any one of claims 1 to 3 wherein X₄ is I.

5. The peptide derivative of any one of claims 1 to 4 wherein X₅ is I.

6. The peptide derivative of any one of claims 1 to 5 wherein θ is Aoc.

7. The peptide derivative of any one of claims 1 to 6 which is 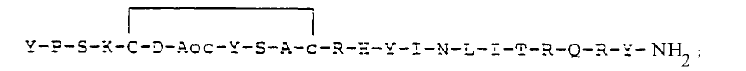 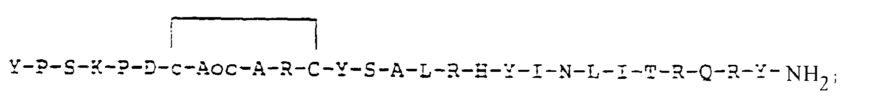 or

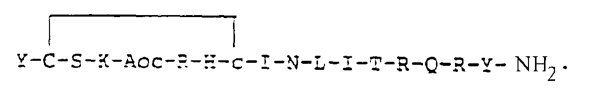

8. A process for preparing a peptide derivative according to any one of claims 1 to 7 which comprises binding a suitably protected tyrosine to an activated resin support, subsequently binding the other alpha amino protected amino acids from proline or cysteine to the carboxy terminal tyrosine to the terminal amino group of the growing peptidic chain which has meanwhile been exposed by removing its amino protecting groups, where an internally cyclized peptide derivative is desired subjecting the linear peptide to an oxidative coupling, and finally isolating the desired peptide or a pharmaceutically acceptable salt thereof.

9. A method for the preparation of a pharmaceutical composition comprising combining peptide derivative or a pharmaceutically acceptable salt thereof according to any one of claims 1 to 7 or a mixture thereof with a pharmaceutically acceptable carrier.

10. A method according to claim 9 wherein said peptide has the formula 3 as defined in claims 1 to 5 and the pharmaceutical composition prepared is useful in the treatment of hypotension.

11. A method according to claim 9 wherein the pharmaceutical composition prepared is useful in the treatment of eating aversion disorders.

12. A method according to claim 9 wherein the pharmaceutical composition prepared is useful as an activator of a neuropeptide Y receptor.

## Patentansprüche (Patentansprüche für folgende(n) Vertragsstaat(en) : AT, BE, CH, DE, FR, GB, IT, LI, LU, NL, SE)

1. Peptidderivat der Formeln 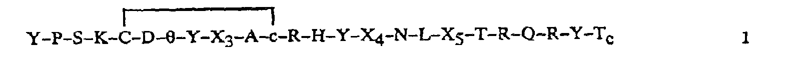 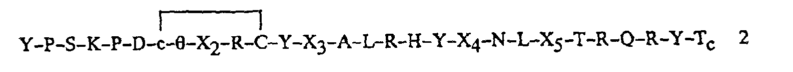 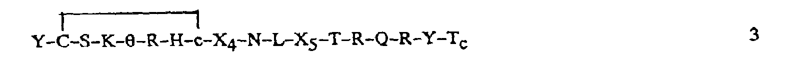 in denen
X₂ S oder A ist;
X₃ S oder A ist;
X₄ L, l, M, Nle oder V ist;
X₅ L, l, M, Nle oder V ist;
Tc OR' oder NHR' ist,
worin R' ein Wasserstoffatom oder ein C₁-C₄-Alkylrest ist:
θ ein Rest der Strukturformel -NH-(CH₂)ₙ-CO₂- ist,
worin n eine ganze Zahl von 1 - 11 bedeutet,
oder ein pharmazeutisch verträgliches Salz davon.

2. Peptidderivat nach Anspruch 1, worin X₂ A ist.

3. Peptidderivat nach Anspruch 1 oder 2, worin X₃ S ist.

4. Peptidderivat nach einem der Ansprüche 1 bis 3, worin X₄ ist.

5. Peptidderivat nach einem der Ansprüche 1 bis 4, worin X₅ l ist.

6. Peptidderivat nach einem der Ansprüche 1 bis 5, worin 8 Aoc ist.

7. Peptidderivat nach einem der Ansprüche 1 bis 6, das 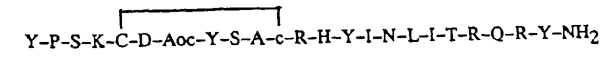 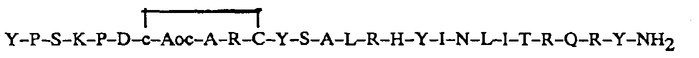 oder 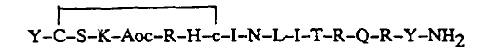 ist.

8. Verfahren zur Herstellung eines Peptidderivats nach einem der Ansprüche 1 bis 7, umfassend das Binden eines geeignet geschützten Tyrosins an einen aktivierten Harzträger, anschließend das Binden der weiteren a-Aminogeschützten Aminosäuren von Prolin oder Cystein bis zum Carboxyl-terminierten Tyrosin an die terminale Aminogruppe der wachsenden Peptidkette, die zwischenzeitlich durch Entfernen ihrer Aminoschutzgruppen freigesetzt worden ist, oxidatives Kuppeln des linearen Peptids, wenn ein intramolekular cyclisiertes Peptid gewünscht ist, und schließlich Isolieren des gewünschten Peptids oder eines pharmazeutisch verträglichen Salzes davon.

9. Peptidderivat oder ein pharmazeutisch verträgliches Salz davon nach einem der Ansprüche 1 bis 7 oder ein Gemisch davon zur Verwendung als Arzneistoff.

10. Peptidderivat der Formel 3 oder ein pharmazeutisch verträgliches Salz davon nach einem der Ansprüche 1 bis 7 oder ein Gemisch davon zur Behandlung von Hypotension.

11. Peptidderivat oder ein pharmazeutisch verträgliches Salz davon nach einem der Ansprüche 1 bis 7 oder ein Gemisch davon zur Behandlung von Eßaversionsstörungen.

12. Peptidderivat oder ein pharmazeutisch verträgliches Salz davon nach einem der Ansprüche 1 bis 7 oder ein Gemisch davon zur Verwendung als Aktivator eines Neuropeptid Y-Rezeptors.

## Patentansprüche (Patentansprüche für folgende(n) Vertragsstaat(en) : ES, GR)

1. Peptidderivat der Formeln 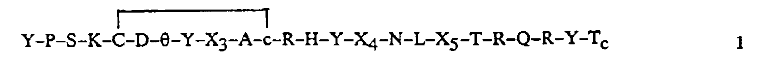 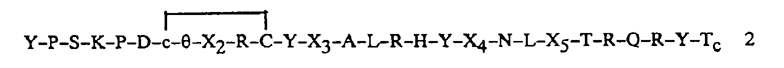 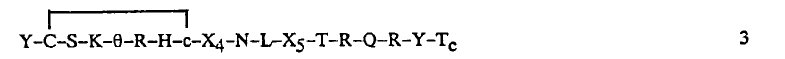 in denen
X₂ S oder A ist;
X₃ S oder A ist;
X₄ L, l, M, Nle oder V ist;
X₅ L, l, M, Nle oder V ist;
Tc OR' oder NHR' ist;
worin R' ein Wasserstoffatom oder ein C₁-C₄-Alkylrest ist;
θ ein Rest der Strukturformel -NH-(CH₂)ₙ-CO₃₂- ist,
worin n eine ganze Zahl von 1 - 11 bedeutet,
oder ein pharmazeutisch verträgliches Salz davon.

2. Peptidderivat nach Anspruch 1, worin X₂ A ist.

3. Peptidderivat nach Anspruch 1 oder 2, worin X₃ S ist.

4. Peptidderivat nach einem der Ansprüche 1 bis 3, worin X₄ l ist.

5. Peptidderivat nach einem der Ansprüche 1 bis 4, worin X₅ l ist.

6. Peptidderivat nach einem der Ansprüche 1 bis 5, worin 8 Aoc ist.

7. Peptidderivat nach einem der Ansprüche 1 bis 6, das 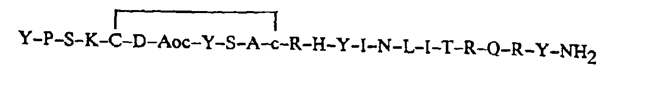 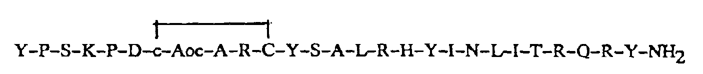 oder 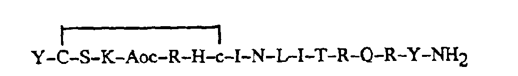 ist.

8. Verfahren zur Herstellung eines Peptidderivats nach einem der Ansprüche 1 bis 7, umfassend das Binden eines geeignet geschützten Tyrosins an einen aktivierten Harzträger, anschließend das Binden der weiteren a-Aminogeschützten Aminosäuren von Prolin oder Cystein bis zum Carboxyl-terminierten Tyrosin an die terminale Aminogruppe der wachsenden Peptidkette, die zwischenzeitlich durch Entfernen ihrer Aminoschutzgruppen freigesetzt worden ist, oxidatives Kuppeln des linearen Peptids, wenn ein intramolekular cyclisiertes Peptid gewünscht ist, und schließlich Isolieren des gewünschten Peptids oder eines pharmazeutisch verträglichen Salzes davon.

9. Verfahren zur Herstellung eines Arzneimittels, das das Kombinieren eines Peptidderivats oder eines pharmazeutisch verträglichen Salzes davon nach einem der Ansprüche 1 bis 7 oder eines Gemisches davon mit einem pharmazeutisch verträglichen Träger umfaßt.

10. Verfahren nach Anspruch 9, worin das Peptid die Formel 3 besitzt, wie in Anspruch 1 bis 5 definiert, und das hergestellte Arzneimittel zur Behandlung von Hypotension nützlich ist.

11. Verfahren nach Anspruch 9, worin das hergestellte Arzneimittel zur Behandlung von Eßaversionsstörungen nützlich ist.

12. Verfahren nach Anspruch 9, worin das hergestellte Arzneimittel als Aktivator eines Neuropeptid Y-Rezeptors nützlich ist

## Revendications (Revendications pour l'(les) Etat(s) contractant(s) suivant(s) : AT, BE, CH, DE, FR, GB, IT, LI, LU, NL, SE)

1. Dérivé peptidique de formule 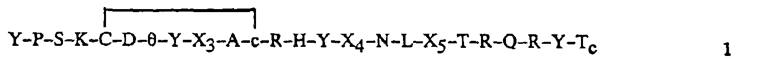 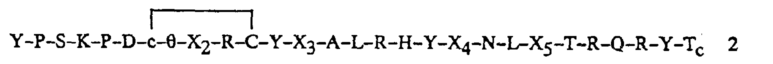 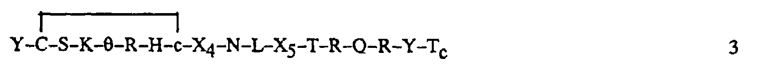 où
X₂ est S ou A
X₃ est S ou A;
X₄ est L, 1, M, Nle, ou V;
X₅ est L, 1, M, Me, Nle, ou V;
Tc est OR' ou NHR'
où R' est un atome d'hydrogène ou un groupement alkyle en C₁-C₄ ;
θ est un groupement de formule développée
-NH-(CH₂)ₙ-C0₂-
où n est un entier entre 1 et 11,
ou un sel pharmaceutiquement acceptable de ce dernier.

2. Dérivé peptidique selon la revendication 1, où X₂ est A.

3. Dérivé peptidique selon la revendication 1 ou 2, où X₃ est S.

4. Dérivé peptidique selon une quelconque des revendications 1 à 3, où X₄ est 1

5. Dérivé peptidique selon une quelconque des revendications 1 à 4, où X₅ est 1.

6. Dérivé peptidique selon une quelconque des revendications 1 à 5, où 8 est Aoc.

7. Dérivé peptique selon une quelconque des revendications 1 à 6, qui est 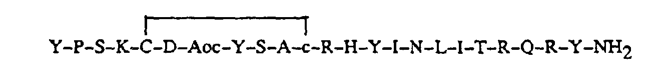 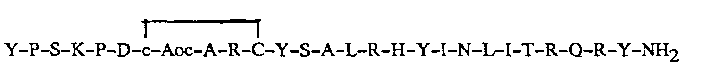 ou

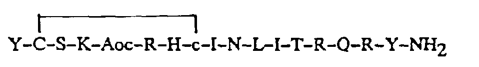

8. Procédé de préparation d'un dérivé peptidique selon une quelconque des revendications 1 à 7, lequel comprend la fixation d'une tyrosine protégée de façon appropriée à un support résineux activé, la fixation subséquente des autres acides aminés protégés en a-amine, de la proline ou la cystéine à la tyrosine carboxyle terminale, au groupement amine terminal de la chaîne peptidique croissante qui a été dans l'intervalle exposée par enlèvement de ses groupements protecteurs d'amine, la soumission du peptide linéaire à un couplage oxydatif lorsqu'un dérivé peptidique cyclisé de façon interne est désiré et finalement l'isolement du peptide désiré ou d'un sel pharmaceutiquement acceptable de ce dernier.

9. Dérivé peptidique ou sel pharmaceutiquement acceptable de ce dernier selon une quelconque des revendications 1 à 7 ou leur mélange pour utilisation comme substance pharmaceutiquement active.

10. Dérivé peptidique de formule 3 ou sel pharmaceutiquement acceptable de ce dernier selon une quelconque des revendications 1 à 7 ou leur mélange pour le traitement de l'hypotension.

11. Dérivé peptidique ou sel pharmaceutiquement acceptable de ce dernier selon une quelconque des revendications 1 à 7 ou leur mélange pour le traitement des troubles de type dégoût pour la nourriture.

12. Dérivé peptidique ou sel pharmaceutiquement acceptable de ce dernier selon une quelconque des revendications 1 à 7 ou leur mélange pour utilisation comme activateur d'un récepteur au neuropeptide Y

## Revendications (Revendications pour l'(les) Etat(s) contractant(s) suivant(s) : ES, GR)

1. Dérivé peptidique de formule 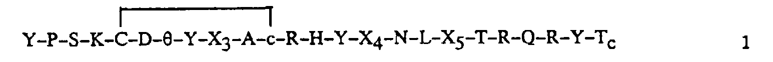 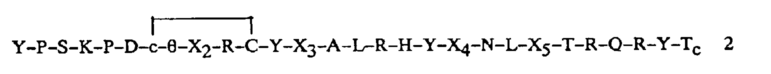 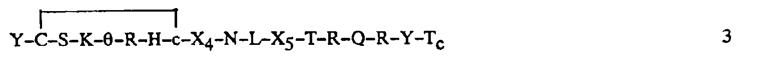 où
X₂ est S ou A
X₃ est S ou A;
X₄ est L, l, M, Nle, ou V ;
X₅ est L, l, M, Nle, ou V ;
Tc est OR' ou NHR'
où R' est un atome d'hydrogène ou un groupement alkyle en C₁-C₄ ;
θ est un groupement de formule développée 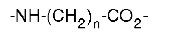
où n est un entier entre 1 et 11,
ou un sel pharmaceutiquement acceptable de ce dernier.

2. Dérivé peptidique selon la revendication 1, où X₂ est A

3. Dérivé peptidique selon la revendication 1 ou 2, où X₃ est S.

4. Dérivé peptidique selon une quelconque des revendications 1 à 3, où X₄ est 1.

5. Dérivé peptidique selon une quelconque des revendications 1 à 4, où Xₛ est 1.

6. Dérivé peptidique selon une quelconque des revendications 1 à 5, où 8 est Aoc.

7. Dérivé peptique selon une quelconque des revendications 1 à 6, qui est 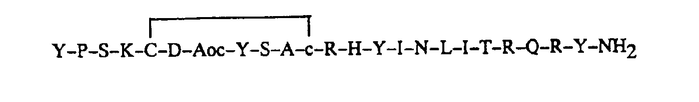 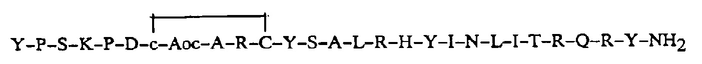 ou

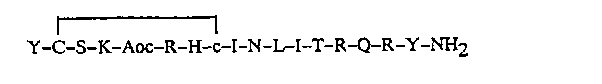

8. Procédé de préparation d'un dérivé peptidique selon une quelconque des revendications 1 à 7, lequel comprend la fixation d'une tyrosine protégée de façon appropriée à un support résineux activé, la fixation subséquente des autres acides aminés protégés en a-amine, de la proline ou la cystéine à la tyrosine carboxyle terminale, au groupement amine terminal de la chaîne peptidique croissante qui a été dans l'intervalle exposée par enlèvement de ses groupements protecteurs d'amine, la soumission du peptide linéaire à un couplage oxydatif lorsqu'un dérivé peptidique cyclisé de façon interne est désiré et finalement l'isolement du peptide désiré ou d'un sel pharmaceutiquement acceptable de ce dernier.

9. Procédé de préparation d'une composition pharmaceutique comprenant l'association d'un dérivé peptidique ou d'un sel pharmaceutiquement acceptable de ce dernier selon une quelconque des revendications 1 à 7 ou de leur mélange avec un véhicule pharmaceutiquement acceptable.

10. Procédé selon la revendication 9, dans lequel ledit peptide présente la formule 3 selon les revendications 1 à 5 et la composition pharmaceutique préparée est utile dans le traitement de l'hypotension.

11. Procédé selon la revendication 9, dans lequel la composition pharmaceutique préparée est utile dans le traitement des troubles de type dégoût de la nourriture.

12. Procédé selon la revendication 9, dans lequel la composition pharmaceutique préparée est utile comme activateur d'un récepteur au neuropeptide Y.
